Europäisches Patentamt

European Patent Office

Office européen des brevets

(11) **EP 3 375 366 B1**

(12) **EUROPÄISCHE PATENTSCHRIFT**

(45) Veröffentlichungstag und Bekanntmachung des Hinweises auf die Patenterteilung:
**17.02.2021 Patentblatt 2021/07**

(51) Int Cl.:
*A61B 5/053* (2021.01)     *A61B 5/00* (2006.01)

(21) Anmeldenummer: **18161691.3**

(22) Anmeldetag: **14.03.2018**

(54) **VORRICHTUNG UND VERFAHREN ZUM ERMITTELN DES HYDRATIONSZUSTANDES EINES KÖRPERS EINES MENSCHEN ODER EINES SÄUGETIERES**

DEVICE AND METHOD FOR DETERMINING THE HYDRATION STATE OF A BODY OF A HUMAN BEING OR OF A MAMMAL

DISPOSITIF ET PROCÉDÉ DE DÉTERMINATION DE L'ÉTAT D'HYDRATATION D'UN CORPS HUMAIN OU MAMMIFÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.03.2017 DE 102017105447**

(43) Veröffentlichungstag der Anmeldung:
**19.09.2018 Patentblatt 2018/38**

(73) Patentinhaber: **Senetics Healthcare Group GmbH & Co. KG**
**91522 Ansbach (DE)**

(72) Erfinder:
- **Wolfgang, Sening**
  **91522 Ansbach (DE)**
- **Wiehl, Michael**
  **91522 Erlangen (DE)**

(74) Vertreter: **Wittmann, Günther**
**Patentanwaltskanzlei Wittmann**
**Frans-Hals-Straße 31**
**81479 München (DE)**

(56) Entgegenhaltungen:
**DE-A1-102008 002 520**     **US-A1- 2008 234 600**
**US-A1- 2009 043 222**     **US-A1- 2015 289 820**

- **MOLL JOCHEN ET AL: "Towards integrated measurements of dielectric tissue properties at microwave frequencies", 2015 9TH EUROPEAN CONFERENCE ON ANTENNAS AND PROPAGATION (EUCAP), EURAAP, 13. April 2015 (2015-04-13), Seiten 1-5, XP033212443, [gefunden am 2015-08-27]**

## Beschreibung

Die vorliegende Erfindung offenbart eine Vorrichtung und ein Verfahren zum Ermitteln des Hydrationszustandes eines Menschen oder eines Säugetieres. Insbesondere ermöglicht die vorliegende Erfindung eine kontinuierliche Überwachung des Hydrationszustandes eines lebendigen Körpers. Die Erfindung eignet sich insbesondere zur kontinuierlichen Überwachung des Flüssigkeitshaushaltes bzw. Hydrationszustandes eines Menschen.

## Stand der Technik

Im Stand der Technik kann eine Messung des Flüssigkeitsgehaltes eines lebendigen Körpers nach momentanem Stand der Wissenschaft direkt oder indirekt durchgeführt werden. Zu dem direkten Messverfahren zählt beispielsweise die Körperwassermessung durch eine radioaktive Isotopverbindung. Ein Vorteil der direkten Messung ist, dass der aktuelle Flüssigkeitsgehalt eines Körpers gemessen werden kann und sind folglich die genauestens zur Verfügung stehenden Mittel, um einen Hydrationszustand zu bestimmen. An diesem Stand der Technik ist nachteilig, dass die meisten direkten Messtechniken die Einnahme und/oder intravenöse Verabreichung einer potenziell gefährlichen Substanz aufweisen. Ferner ist an diesem Stand der Technik nachteilig, dass aufgrund der erforderlichen umfangreichen Analysen, beispielsweise von Blutproben, die Messung des Hydrationszustandes nicht kontinuierlich durchgeführt werden kann.

Beispiel indirekter Verfahren sind die Ultraschallmessung und Spektroskopie. Bei diesem Verfahren ist nachteilig, dass der Nachweis des gesamten Körperwassergehaltes schwierig ist.

Die Ultraschallmessung nützt die Abhängigkeit der Ausbereitungsgeschwindigkeit des Ultraschallsignals in einer muskulären bzw. molekularen Zusammensetzung. Mit diesem Verfahren kann lediglich der lokale Wassergehalt eines Muskels gemessen werden. Nachteilig ist auch, dass das Ergebnis nicht zwangsläufig mit dem Feuchtigkeitsgehalt des Körpers korreliert werden kann. Dieser Stand der Technik ist beispielsweise in der US 7,291,190 und der US 7,033,321 beschrieben.

Die Spektroskopie basiert auf der Berechnung der Absorption elektromagnetischer Strahlung durch die Haut, die eine Korrelation mit dem Wasserhaushalt ermöglicht. An diesem Verfahren ist nachteilig, dass der Feuchtigkeitsgehalt bzw. Flüssigkeitsgehalt der Haut nicht mit dem Flüssigkeitsgehalt des ganzen Körpers in Verbindung gesetzt werden kann.

Die DE 10 2014 109 549 A1 offenbart einen in den Körper zu implantierenden Sensor, der in vivo einen interstitiellen Flüssigkeitsparameter ermitteln kann. An diesem Stand der Technik ist nachteilig, dass der Sensor in den Körper implantiert werden muss, was in den meisten Fällen unerwünscht ist und die Anzahl der Anwendungen einschränkt.

Die US 2009/0043222 A1 offenbart in der Zusammenfassung die Ermittlung des Niveaus einer extrazellulären Flüssigkeit mittels Bestimmung eines Impedanzwertes. Die Impedanz und Admittanz werden über einen großen Frequenzbereich ermittelt.

Die US 2015/0289820 A1 betrifft die Messung des Hydrationszustandes mittels Impedanzmessung.

Die DE 10 2008 002 520 A) offenbart eine Messung der Eigenschaften der Haut mittels elektrischer Ströme, um die Hydration zu messen.

Derzeit ist in der stationären und/oder ambulanten Pflege kein Verfahren bekannt, mit dem zum einen der aktuelle Zustand der Flüssigkeitsversorgung eines Patienten ermittelt werden kann und zum anderen eine angemessene Dokumentation des Flüssigkeitszustandes eines Patienten durchgeführt werden kann. Da die Verfahren des Standes der Technik lediglich ungenaue Ergebnisse zur Verfügung stellen und die Anforderung an die Ermittlung der Flüssigkeitsversorgung des Körpers eines Patienten sehr hoch sind, ist es professionellen Dienstleistern und professionellen medizinischen Einrichtungen nur bedingt möglich, den Zustand der Flüssigkeitsversorgung eines Patienten adäquat zu erfassen und zu dokumentieren.

Die Überwachung des Flüssigkeitshaushaltes ist insbesondere bei der Pflege älterer Menschen, bei Sportlern, bei Menschen, die hauptsächlich im Büro arbeiten, bei Fahrern eines Kraftfahrzeuges, bei der Überwachung des Wasserhaushalts von Tieren und beim Messen der Feuchte von Hautersatzstoffen wichtig. Trotz der Tatsache, dass die Überwachung des Wasserhaushaltes bzw. des Hydratisierungsgrades für viele Personen wichtig ist, ist zurzeit kein zuverlässiges Verfahren bekannt, mit dem die Überwachung von Änderungen des Hydrationszustandes mit einem angemessenen Aufwand und einer angemessenen Zuverlässigkeit erfolgen kann. Die im Stand der Technik verfügbaren Verfahren sind entweder mittelbare Verfahren oder ungenau. Die direkten Verfahren sind aufwändig und komplex und mit einer Einnahme unerwünschter Mittel verbunden.

## Kurze Beschreibung der Erfindung

Die Erfindung stellt sich zur Aufgabe, den Hydrationszustand eines Körpers eines Menschen oder Säugetieres mit einfachen Mitteln kontinuierlich zu überwachen.

Die Aufgabe der Erfindung wird durch eine Hydrationszustanderfassungsvorrichtung nach Anspruch 1, durch ein Verfahren zum Ermitteln des Hydrationszustandes nach Anspruch 7 und durch ein Computerprogrammprodukt nach Anspruch 12 gelöst. Die abhängigen Ansprüche beanspruchen bevorzugte Ausführungsformen.

Eine erfindungsgemäße Hydrationszustanderfassungsvorrichtung, die dazu ausgebildet ist, den Hydrationszustand eines Körpers eines Menschen oder eines Säugetieres oder eines beliebigen Organismus oder Gewebes zu erfassen, umfasst eine erste Sensoranordnung, eine zweite Sensoranordnung und eine Auswerteeinrichtung. Die erste Sensoranordnung ist dazu ausgebildet, elektromagnetische Wellen über die Haut in den Körper zu emittieren und dazu ausgebildet, vom Körper transmittierte elektromagnetische Wellen und/oder vom Körper reflektiere elektromagnetische Wellen zu erfassen. Die erste Sensoranordnung kann alternativ oder zusätzlich dazu ausgebildet sein, die Impedanz des Körpers zu messen. Die erste Sensoranordnung ist dazu ausgebildet, auf der Haut des Körpers angeordnet zu werden. Die erste Sensoranordnung ermittelt zumindest eine erste Messgröße, die auf den transmittierten und/oder reflektierten elektromagnetischen Wellen beruht. Die zweite Sensoranordnung ist dazu ausgebildet, zumindest eine weitere physikalische Größe des Körpers zu messen und eine zweite Messgröße auszugeben, die auf der zweiten physikalischen Größe beruht. Die Auswerteeinrichtung ist dazu ausgebildet, auf Grundlage der ersten Messgröße und der zweiten Messgröße den Hydrationszustand des Körpers zu ermitteln.

Bei einer anderen Ausführungsform kann die erste Sensoranordnung ein Bioimpedanzsensor ein. Bei der Bioimpedanzmessung wird eine elektrische Impedanz des Körpers durch zwei Elektroden auf dem Körper bei unterschiedlichen Frequenzen ermittelt. Mittels niedriger Frequenzen kann der extrazelluläre Wassergehalt ermittelt werden, und mittels höherer Frequenzen kann der gesamte Wassergehalt im Messbereich ermittelt werden. Die erste Sensoranordnung misst die Resonanzfrequenz des Bereichs des Körpers, in dem sich die von der ersten Sensoranordnung emittierten elektromagnetischen Wellen ausbreiten.

Bei einer Ausführungsform kann die erste Sensoranordnung die Feuchtigkeit des Gewebes bzw. des Körpers mittels einer Reflexion elektromagnetischer Wellen ermitteln. Bei dieser Ausführungsform werden die elektromagnetischen Wellen von einer Seite des Körpers in das Gewebe eingestrahlt. Durch die Reflexion am Gewebe des Körpers, werden die elektromagnetischen Wellen wieder an dem Sensor ermittelt und als Messgröße für die Auswertung verwendet. In diesem Fall ist lediglich eine Komponente erforderlich, die gleichzeitig als Sender und Empfänger arbeitet. Diese Komponente berührt die Haut des Körpers aber wird nicht in diesen implantiert.

Bei einer zweiten Ausführungsform kann mittels der ersten Sensoranordnung die Messung der Gewebefeuchte mittels Transmission erfolgen. In diesem Fall werden elektromagnetische Wellen von einer Seite in das Gewebe des Körpers mittels eines Senders eingestrahlt. Die elektromagnetischen Wellen werden auf der dem Sender gegenüber liegenden Seite von einer Detektionseinrichtung empfangen. Aufgrund der Reflexion am Gewebe werden die elektromagnetischen Wellen wieder am Sensor ermittelt und durch einen darin angeordneten Empfänger erfasst. Zusätzlich werden die elektromagnetischen Wellen, die das Gewebe des Körpers durchlaufen haben, an der Detektionseinrichtung erfasst. Bei dieser Ausführungsform können sowohl die reflektierten als auch die transmittierten elektromagnetischen Wellen berücksichtigt werden.

Die erfindungsgemäße Hydrationszustanderfassungsvorrichtung benutzt mehrere Sensoranordnungen, um den Hydrationszustand zu messen. Dadurch ist es möglich, den Hydrationszustand des Körpers kontinuierlich mit einer ausreichenden Genauigkeit zu messen.

Die vorliegende Erfindung hat den Vorteil, dass Sie kompakt aufgebaut werden kann und als Überwachungseinrichtung von einem Patienten mitgeführt werden kann. Ferner wird der Feuchtigkeitshaushalt eines Patienten kontinuierlich überwacht, ohne dass aufwändige Verfahren durchgeführt werden müssen. Ferner muss kein Sensor in den Körper implantiert werden. Zusätzlich müssen keine potentiell gefährlichen Mittel durch den Patienten eingenommen werden.

Die erfindungsgemäße Hydrationszustanderfassungsvorrichtung erzielt eine höhere Genauigkeit als vergleichbare Vorrichtungen des Standes der Technik, weil eine Mehrzahl unterschiedlicher physikalischer Größen ermittelt bzw. überwacht wird. Die Hydrationszustanderfassungsvorrichtung kann zumindest eine weitere Sensoranordnung aufweisen, die je dazu ausgebildet sind, zumindest eine weitere physikalische Größe des Körpers zu messen und die je eine weitere Messgröße ausgeben, die auf der weiteren physikalischen Größe beruht. Die Auswerteeinrichtung kann dazu ausgebildet sein, auf Grundlage der ersten Messgröße, der zweiten Messgröße und der weiteren Messgrößen den Hydrationszustand des Körpers zu ermitteln. Je mehr physikalische Größen ermittelt bzw. überwacht werden, desto genauer kann der Hydrationszustand des Körpers ermittelt werden.

Bei einer Ausführungsform kann die erste Sensoranordnung die Resonanzfrequenz des Bereichs des Körpers, in dem sich die elektromagnetischen Wellen von der ersten Sensoranordnung ausbreiten, messen.

Die zweite und/oder weitere Sensoranordnung kann dazu ausgebildet sein, eine Hautfeuchte, eine Sauerstoffsättigung, einen Puls, einen Blutdruck, einen Körperfettanteil zu messen. Dadurch lässt sich der Hydrationszustand des Körpers genauer ermitteln.

Die Auswerteeinrichtung kann dazu ausgebildet sein, den Hydrationszustand des Körpers mittels der folgenden Formeln zu ermitteln:

$$TBW = K_0 + K_1 \times f_1(MG_1, MG_{1ref}) + \ldots + f_n(MG_n, MG_{nref});$$

Die Auswerteeinrichtung kann dazu ausgebildet sein, den Hydrationszustand des Körpers mittels der folgenden Formeln zu ermitteln, falls lediglich zwei Messgrößen verwendet werden:

$$TBW = K_1 + K_2 \times MG_1/MG_{1ref} + K_3 \times MG_2/MG_{2ref};$$

Falls mehr als zwei Sensoranordnungen verwendet werden, kann der Hydrationszustand des Körpers mittels der folgenden Formel ermittelt werden:

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref} \ldots;$$

wobei gilt:

TBW: $m_{Wasser}/m_{Körper}$;
MGx: Messgröße x;
$MG_{xref}$: Referenzwert der Messgröße x;
$f_1(MG_1, MG_{1ref})$ eine beliebige Funktion der Messgröße 1 und deren Referenzwert ist; und
$f_n(MG_n, MG_{nref})$ eine beliebige Funktion der Messgröße n und deren Referenzwert ist. Bei einer andere Ausführungsform kann ein Summand eine beliebige Funktion sein, beispielsweise $K_n \times f(MG_n, MG_{nref})$, wobei $f(x, xref)$ ist eine beliebige Funktion ist, beispielsweise $MG_n/MG_{nref}$, $MG_n/MG_{nref} \times \exp(MG_n + K_n)$, wobei $K_n$ eine Konstante ist, oder $f(x, xref)$ ist eine logarithmische Funktion.

Die Hydrationszustandserfassungsvorrichtung kann eine Kalibriereinrichtung aufweisen, die dazu ausgebildet ist, den Referenzwert $MG_{xref}$ einer Messgröße MGx durch Mittelwertbildung über alle in einer Trainingsphase (Kalibrierungsphase), die einen vorbestimmten Zeitraum dauert, erfassten Messgrößen MGx einer physikalischen Größe zu ermitteln.
Die Mittelwerte werden für alle Messgrößen über den vorbestimmten Zeitraum der Trainingsphase erfasst. Die Mittelwerte dienen zum Normieren der Messwerte. Die Trainingsphase kann bei einem Menschen beispielsweise etwa eine Woche bis etwa drei Wochen dauern. Zusätzlich wird der Wert für den idealen Hydrationszustand TBW (Total Body Water; Gesamtanteil des Wassers im Körper) mittels der Formel nach Watson, Hume-Weyer, und/oder Mellits-Cheek ermittelt.
Der Wert TBW wird mittels folgender Formel berechnet $TBW = m_{Wasser}/m_{Körper}$.
Berechnung nach Watson:

Männer,

$$TBW = 2.447 - (0.09156 \times age) + (0.1074 \times h) + (0.3362 \times m_{Body});$$

Frauen,

$$TBW = -2.097 + (0.1069 \times h) + (0.2466 \times m_{Body});$$

Berechnung nach Hume-Weyers :

Männer,

$$TBW = (0.194786 \times h) + (0.296785 \times m_{Body}) - 14.012934;$$

Frauen,

$$TBW = (0.34454 \times h) + (0.183809 \times m_{Body}) - 35.270121;$$

Mellits-Cheek (Kinder):

Jungen,

$$TBW = -1.927 + 0.465 \times m_{Body} + 0.045 \times h \text{ bei } h < 132.7 \text{ cm};$$

Jungen,

$$TBW = -21.993 + 0.406 \times m_{Body} + 0.209 \times h \text{ bei } h > 132.7 \text{ cm};$$

Mädchen,

$$TBW = 0.076 + 0.507 \times m_{Body} + 0.013 \times h \text{ bei } h < 110.8 \text{ cm};$$

Mädchen,

$$TBW = -10.313 + 0.252 \times m_{Body} + 0.154 \times h \text{ bei } h > 110.8 \text{ cm};$$

Der Wert TBW hängt Vom Alter, Geschlecht und Gewicht eines Nutzers ab. Typischerweise beträgt dieser Wert zwischen etwa 50 % bis etwa 60 % bei einem erwachsenen Menschen. Während der Trainingsphase wird der mittlere TBW des jeweiligen Nutzers bestimmt.

Die zuvor beschriebenen Konstanten $K_1$, $K_2$, $K_3$, $K_4$, $K_5$ etc. werden mittels einer Studie über eine Mehrzahl Menschen bzw. Tiere ermittelt.

Das erfindungsgemäße Verfahren kann eine Mehrzahl Werte der Messgrößen über einen vorbestimmten Zeitraum, d.h. Trainingszeitraum (Kalibrierzeitraum), ermitteln. Das Verfahren kann einen Referenzwert einer Messgröße durch Ermitteln eines Durchschnittswertes der jeweiligen Messgröße auf Grundlage der im vorbestimmten Zeitraum ermittelten Werte der jeweiligen Messgröße ermitteln.

Die Erfindung offenbart auch ein Computerprogrammprodukt, das, wenn es in einen Speicher eines Computers mit einem Prozessor geladen wird, die Schritte des zuvor beschriebenen Verfahrens ausführt.

Es versteht sich, dass der Computer eine Steuerungseinrichtung, beispielsweise ein sogenannter Controller sein kann. Die Auswerteeinrichtung und die Kalibriereinrichtung können durch den Computer implementiert werden. Geeignete Computer sind dem Fachmann bekannt und müssen hierin nicht weiter beschrieben werden.

Die Erfindung wird nun unter Bezugnahme auf die beigefügten Figuren, die nicht beschränkende Ausführungsformen der Erfindung zeigen, detaillierter beschrieben, wobei:

Figur 1 eine schematische Darstellung einer erfindungsgemäßen Hydrationszustandserfassungsvorrichtung ist;

Figur 2 eine weitere Ausführungsform der Erfindung zeigt;

Figur 3 eine Ausführungsform mit einem Sensorpflaster zeigt; und

Figur 4 eine Ausführungsform zeigt, bei der der Sensor in einer Einrichtung angeordnet ist, die von einem Patienten berührt wird.

Die erfindungsgemäße Hydrationserfassungsvorrichtung 100 umfasst eine Prozessoreinrichtung, beispielsweise einen Miniaturcomputer, der von einem Akkumulator 104, an den eine Ladeeinrichtung 106 angeschlossen ist, mit Strom versorgt wird. An die Prozessoreinrichtung ist ein Bus 108 angeschlossen, beispielsweise ein so genannter CAN-Bus oder SPI-Bus. An den Bus ist eine erste Sensoranordnung 110 angeschlossen, die eine elektromagnetische Welle in den Körper 126 eines Menschen sendet und die Reflexion der elektromagnetischen Welle vom Körper 126 ermittelt. Die erste Sensoranordnung 110 berührt lediglich die Haut des Patienten und wird nicht implantiert. Die Sensoranordnung 110 kann im Wesentlichen den in der DE 10 2014 109 549 A1 beschrieben Sensor aufweisen. Die erste Sensoranordnung gibt ein Signal im Bereich von 250 MHz bis etwa 3,5 THz ab.

Bei einer anderen Ausführungsform kann die erste Sensoranordnung eine sogenannter Bioimpedanzsensor sein, der mittels zumindest zwei Elektroden den Leitwert des Körpers ermittelt, wobei an die Elektroden Ströme mit unterschiedlicher Frequenz angelegt werden..

An den Bus 104 ist ferner ein Hautleitwertsensor 112, ein Pulssensor 114, ein Sensor zum Messen der Sauerstoffsättigung 116 (SpO2-Sensor), ein Sensor 118 zum Ermitteln der Luftfeuchtigkeit und der Temperatur, ein Gyroskop 120 mit Beschleunigungssensoren (Schrittzähler, Lagesensor) und/oder ein Temperatursensor 122 angeschlossen.

Ziel der Hydrationszustandserfassungsvorrichtung 100 ist, den Hydrationszustand eines Patienten zu messen. Typischerweise wird der Hydrationszustand mittels des Wertes TBW ermittelt, wobei gilt, TBW = $m_{Wasser}/m_{Körper}$.

Zur Berechnung des idealen TBW stehen die folgender Formeln zur Verfügung:

Die Auswerteeinrichtung kann dazu ausgebildet sein, den Hydrationszustand des Körpers mittels einer der folgenden Formeln zu ermitteln:

$$TBW = K_0 + K_1 \times f_1(MG_1, MG_{1ref}) + \ldots + f_n(MG_n, MG_{nref});$$

oder

$$TBW = K_1 + K_2 \times MG_1/MG_{1ref} + K_3 \times MG_2/MG_{2ref};$$

oder

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref} \ldots;$$

wobei gilt:

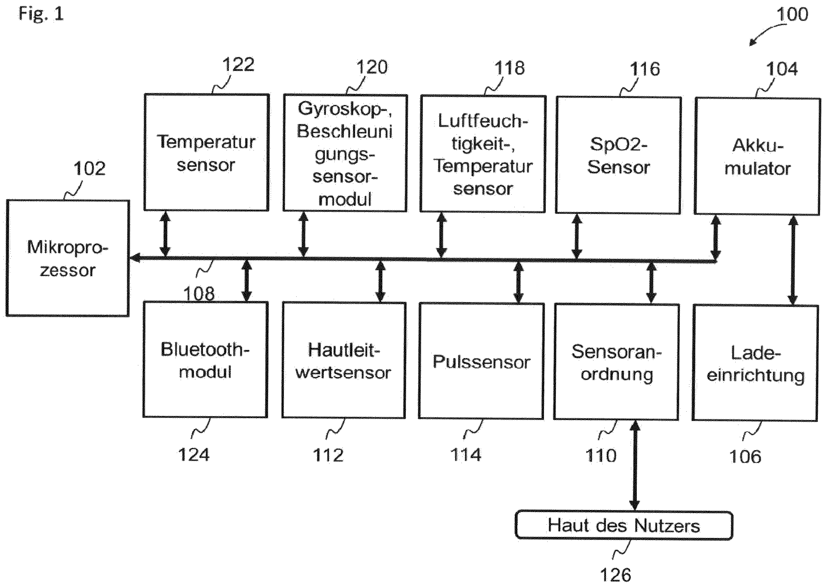

TBW: $m_{Wasser}/M_{Körper}$;
MGx: Messgröße x;
$MG_{xref}$: Referenzwert der Messgröße x;
$f_1(MG_1, MG_{1ref})$ eine beliebige Funktion der Messgröße 1 und deren Referenzwert ist; und
$f_n(MG_n, MG_{nref})$ eine beliebige Funktion der Messgröße n und deren Referenzwert ist. Bei der in Figur 1 gezeigten Ausführungsform ermittelt die erste Sensoranordnung (elektromagnetischer Sensor) eine Resonanzfrequenz f (in Hz), die Dämpfung s (einheitenlos) und/oder die Phase a (in Grad) in dem Bereich des Körpers, den die elektromagnetischen Wellen, die von der ersten Sensoranordnung emittiert werden, durchlaufen. Die Dämpfung und die Phasenverschiebung werden bei der Resonanzfrequenz ermittelt. Der Hautleitwertsensor 112 ermittelt die Hautfeuchte rh (in Prozent). Der Sauerstoffsättigungssensor 116 ermittelt die Sauerstoffsättigung (beispielsweise in %). Der Pulssensor ermittelt den Puls p (in bpm).

Die von der ersten Sensoranordnung emittierten elektromagnetischen Wellen können beispielsweise Haut und/oder Muskelfasern und/oder Knochen und/oder Gefäße und/oder Fettgewebe und/oder übrige Körperbestandteile. durchlaufen, wenn die erste Sensoranordnung am Arm angeordnet wird. Bei einer anderen Ausführungsform können von der ersten Sensoranordnung emittierten elektromagnetischen Wellen beispielsweise Haut und/oder Muskelfasern und/oder Knochen und/oder Gefäße und/oder Fettgewebe und/oder andere Körperbestandteile durchlaufen, wenn die erste Sensoranordnung am Bein, am Rücken, an der Brust oder an übrigen Körperteilen angeordnet ist.

Diese Werte können zyklisch über den Tag bzw. während der Nutzung aufgenommen werden. Je nach Anwendungsfall können die Werte einmal pro Stunde, einmal pro Minute, einmal pro Sekunde oder dergleichen aufgenommen werden. Während der ersten Phase des Verwendens der Hydrationszustandserfassungsvorrichtung durch einen Träger, d. h. Trainingsphase, werden so genannte Referenzwerte ermittelt. Ferner werden Gewichtungsfaktoren ($K_1$, $K_2$, $K_3$, $K_4$, $K_5$, $K_6$, $K_7$) auf Basis der gemessenen Daten, dem mittleren Gewicht, der Größe h und dem Alter A und dem Geschlecht an den Nutzer mittels der zuvor beschriebenen Formeln zum Berechnen des idealen BTW verwendet.

Die Trainingsphase kann bei einem Beispiel etwa ein bis drei Wochen dauern. Während der Trainingsphase werden sogenannte Referenzwerte ermittelt, ein Nutzerprofil erstellt und Übungen durchgeführt. Zur Kalibration bzw.

während des Trainings können in der Trainingsphase definierte Bewegungsabläufe, beispielsweise ausgewählte sportliche Übungen, durchgeführt werden. Damit kann ermittelt werden, wie sich Puls oder Gewebefeuchte bei sportlicher Betätigung ohne signifikanter Dehydration verändern. Die Testphase kann etwa eine Woche dauern. Daran schließt sich eine Nutzungsphase an, in der die Werte für den Hydrationszustand abgelesen werden. Sporadisch werden die abgelesenen Werte mit Messungen von externen Sensoren abgeglichen.

Während der Trainingsphase (Kalibrierungsphase) können die Referenzwerte beispielsweise durch Mittelwertbildung über alle gemessenen Werte bestimmt werden.

$$f_{ref} = \frac{1}{N} \sum_{k=0}^{N-1} \mathrm{f}[\mathrm{k}]$$

Nach Abschluss der Trainingsphase werden die von den Sensoren gemessenen Werte über eine sogenannte Sensordatenfusion miteinander verrechnet. Zuerst wird jeder von den Sensoren ausgegebene Messwert (Messgröße) mit dem entsprechenden Referenzwert normiert. Der resultierende Wert wird mittels des Gewichtungsfaktors gewichtet. Dadurch lässt sich der Wasserhaushalt (Wasseranteil im Körper) prozentual (relativ) mittels folgender Formel berechnen:

$$TBW = \frac{m_{Water}}{m_{Body}}$$

$$TBW = K_1 + K_2 \frac{f}{f_{ref}} + K_3 \frac{rh}{rh_{ref}} + K_4 \frac{SpO2}{SpO2_{ref}} + K_5 \frac{p}{p_{ref}} + K_6 \frac{s}{s_{ref}} + K_7 \frac{a}{a_{ref}}$$

wobei $K_1$ ein erster Gewichtungsfaktor ist;

$K_2$ ein zweiter Gewichtungsfaktor ist; f die gemessene Resonanzfrequenz ist;
f die von der ersten Sensoranordnung erfasse Frequenz ist;
$f_{ref}$ der Referenzwert Resonanzfrequenz ist;
$K_3$ der dritte Gewichtungsfaktor ist;
rh die relative Hautfeuchtigkeit ist;
$rh_{ref}$ der Referenzwert der relativen Hautfeuchtigkeit ist;
$K_4$ ein vierter Gewichtungsfaktor ist;
SpO2 der vom Sensor gemessene Wert der Sauerstoffsättigung ist;
$SpO2_{ref}$ der Referenzwert der Sauerstoffsättigung ist;
$K_5$ ein fünfter Gewichtungsfaktor ist;
p der vom Sensor erfasste Wert des Pulses ist; und
$p_{ref}$ der Referenzwert des Pulses ist.
$K_6$ ein sechster Gewichtungsfaktor ist;
s der vom Sensor erfasste Wert der Dämpfung ist; und
$s_{ref}$ der Referenzwert der Dämpfung ist.
$K_7$ ein siebter Gewichtungsfaktor ist;
a der vom Sensor erfasste Wert der Phase ist; und
$a_{ref}$ der Referenzwert der Phase ist.

Der TBW hängt vom Alter, Geschlecht und Gewicht eines Nutzers ab. Typischerweise liegt dieser Wert zwischen 50 und 60 % bei einem Erwachsenen. Während der so genannten Trainingsphase wird der mittlere TBW des jeweiligen Nutzers bestimmt. Folglich ist die erfindungsgemäße Hydrationszustandserfassungsvorrichtung in der Lage, eine Unterschreitung des zulässigen Hydrationszustandes zu erkennen.

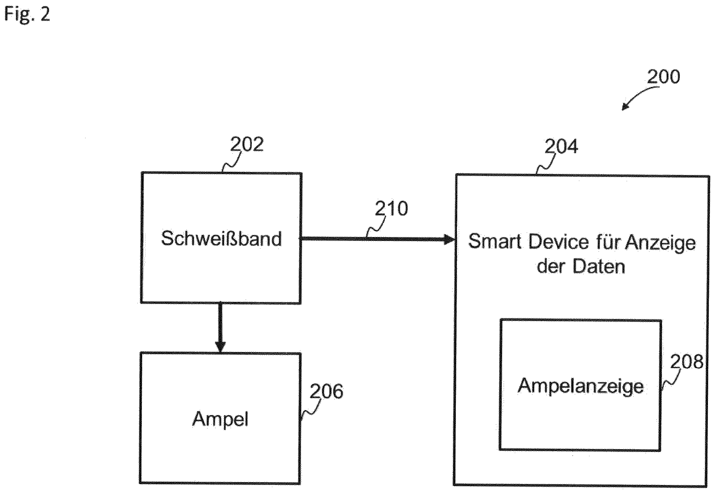

Figur 2 zeigt eine zweite Ausführungsform 200 der Erfindung. In dem Schweißband 202 ist die Hydrationszustandserfassungsvorrichtung 100 gemäß Figur 1 implementiert. Das Schweißband 202 umfasst ferner eine Ampel 206. Leuchtet die Ampel grün, liegt keine Dehydration vor. Zeigt die Ampel orange, liegt eine milde Dehydration vor. Dies bedeutet, dass der Wert TBW um mehr als 2 % von dem in der Trainingsphase ermittelten Mittelwert abgesunken ist. Zeigt die Ampel rot, ist der Wert TBW um mehr als 5 % unter dem während der Trainingsphase ermittelten Mittelwert abgesunken. In diesem Fall liegt eine moderate Dehydration vor.

Die gemessenen und berechneten Werte können über eine Funkverbindung, beispielsweise RFID, Bluetooth an ein anderes Gerät, beispielsweise ein intelligentes Telefon (Smartphone), einen Laptop, einen tragbaren Computer oder dergleichen übertragen werden. Folglich kann der Nutzer bzw. eine Pflegefachkraft den Verlauf der Dehydration verfolgen und dokumentieren.

Es versteht sich, dass bei einer weiteren Ausführungsform weitere Sensoren vorhanden sein können, beispielsweise zur Messung des Blutdrucks, eine Körperfettwaage oder dergleichen.

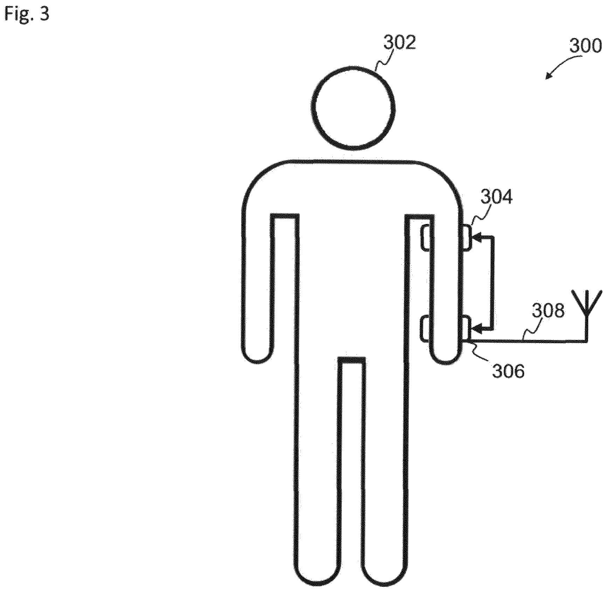

Figur 3 zeigt eine dritte Ausführungsform 300 der Erfindung. Hydrationszustandserfassungsvorrichtung 100 gemäß Figur 1 ist in ein Sensorpflaster 304 integriert, das an der Schulter eines Patienten angebracht ist. Am Handgelenk trägt der Patient 302 ein Armband 306 mit einer Auswerteeinrichtung und einer Anzeigeeinrichtung. Optional kann die Anzeigeeinrichtung eine Schnittstelle für eine Mobilfunkverbindung aufweisen, um über eine Antenne 308 die gemessenen Hydrationszustände an einen anderen Computer weiterzuleiten.

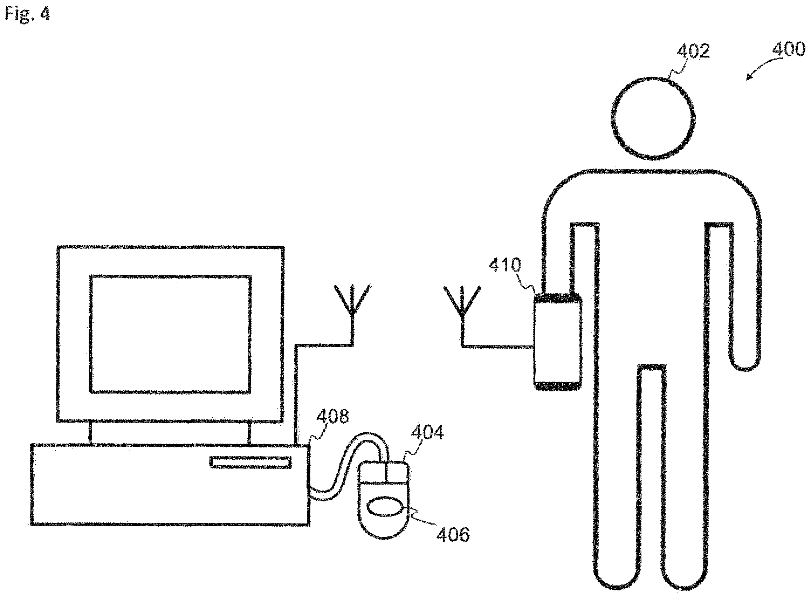

Es wird auf Figur 4 Bezug genommen, die eine vierte Ausführungsform der Erfindung zeigt. Ein Patient 402 berührt mit seiner Hand das in einer Maus 404 eines Computers 408 angebrachten Sensoreinrichtung 406. Die Sensoreinrichtung 406 kann eine Mehrzahl Sensoren aufweisen, die zuvor unter Bezugnahme auf die erste Ausführungsform (Figur 1 beschrieben wurden. Die Maus 404 kann die von den Sensoren der Sensoreinrichtung 406 erfassten Werte an den Computer 408 übertragen, der die von den Sensoren erfassten Werte an eine intelligente Einrichtung 410 (Smartphone oder dergleichen) überträgt, wo die von den Sensoren 406 erfassten Messgrößen ausgewertet werden wie zuvor unter Bezugnahme auf Figuren 1 bis 3 beschrieben wurde.

Die Sensoreinrichtung kann an anderen Objekten angeordnet sein, beispielsweise einem Lenkrad eines Kraftfahrzeuges.

Die Erfindung schafft eine nicht invasive Vorrichtung zum Erfassen des Hydrationszustandes eines Patienten mit einer hohen Genauigkeit. Es ist nicht erforderlich, dass Sensoren in den Patienten implantiert werden. Ferner ist keine aufwändige Diagnostik erforderlich und der Patient muss keine potentiell gefährlichen Mittel einnehmen.

**Patentansprüche**

1. Hydrationszustandserfassungsvorrichtung (100, 200, 300, 400), die dazu ausgebildet ist, den Hydrationszustand eines Körpers eines Menschen oder eines Säugetieres zu erfassen, aufweisend:

   - eine erste Sensoranordnung (110, 304, 406), die dazu ausgebildet ist, Mikrowellen über die Haut in den Körper zu emittieren, und dazu ausgebildet ist, vom Körper transmittierte Mikrowellen und/oder vom Körper reflektiere Mikrowellen zu erfassen, wobei die erste Sensoranordnung dazu ausgebildet ist, auf der Haut des Körpers angeordnet zu werden und wobei die erste Sensoranordnung zumindest eine erste Messgröße ausgibt, die auf der transmittierten und/oder reflektierten Mikrowellen beruht;
   - eine zweite Sensoranordnung (104, 106, 110, 112, 114, 116, 118, 120, 122, 124), die dazu ausgebildet ist, zumindest eine zweite physikalische Größe des Körpers zu messen und eine zweite Messgröße auszugeben, die auf der zweiten physikalischen Größe beruht; und
   - eine Auswerteeinrichtung (102), die dazu ausgebildet ist, auf Grundlage der ersten Messgröße und der zweiten Messgröße den Hydrationszustand des Körpers zu ermitteln,

   wobei die erste Sensoranordnung (110) dazu ausgebildet ist, die Phasenverschiebung und/oder die Dämpfung der Mikrowellen bei einer Resonanzfrequenz des Bereichs des
   Körpers zu messen, in dem sich die von der ersten Sensoranordnung emittierten Mikrowellen ausbreiten.

2. Hydrationserfassungsvorrichtung (100, 200, 300, 400) nach Anspruch 1, wobei die erste Sensoranordnung (110, 304, 406), dazu ausgebildet ist, die Impedanz des Körpers zu erfassen.

3. Hydrationszustandserfassungsvorrichtung (100, 200, 300, 400), nach Anspruch 1 oder 2, aufweisend zumindest eine weitere Sensoranordnungen (104, 106, 110, 112, 114, 116, 118, 120, 122, 124), die je dazu ausgebildet sind, zumindest eine weitere physikalische Größe des Körpers zu messen und die je eine weitere Messgröße ausgeben, die auf der weiteren physikalischen Größe beruht, wobei die Auswerteeinrichtung, dazu ausgebildet ist, auf Grundlage der ersten Messgröße, der zweiten Messgröße und der weiteren Messgröße den Hydrationszustand des Körpers zu ermitteln.

4. Hydrationszustandserfassungsvorrichtung (100, 200, 300, 400) nach einem der Ansprüche 1 bis 3, wobei die zweite und/oder weitere Sensoranordnung (104, 106, 110, 112, 114, 116, 118, 120, 122, 124) dazu ausgebildet sind, je zumindest eine der folgenden physikalischen Größen zu messen:

- eine Hautfeuchte;
- eine Sauerstoffsättigung;
- einen Puls;
- einen Blutdruck;
- einen Körperfettanteil.

5. Hydrationszustandserfassungsvorrichtung (100, 200, 300, 400) nach einem der Ansprüche 1 bis 4, wobei die Auswerteeinrichtung dazu ausgebildet ist, den Hydrationszustand des Körpers mittels zumindest einer der folgenden Formeln zu ermitteln:

$$TBW = K_0 + K_1 \times f_1(MG_1, MG_{1ref}) + \ldots + f_n(MG_n, MG_{nref});$$

oder

$$TBW = K_1 + K_2 \times MG_1/MG_{1ref} + K_3 \times MG_2/MG_{2ref};$$

oder

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref} \ldots;$$

wobei gilt:

TBW: $m_{Wasser}/m_{Körper}$;
$MG_x$: Messgröße x;
$MG_{xref}$: Referenzwert der Messgröße x;
$f_1(MG_1, MG_{1ref})$ eine beliebige Funktion der Messgröße 1 und deren Referenzwert ist;
und
$f_n(MG_n, MG_{nref})$ eine beliebige Funktion der Messgröße n und deren Referenzwert ist.

6. Hydrationszustandserfassungsvorrichtung nach einem der Ansprüche 1 bis 5, ferner aufweisend eine Kalibriereinrichtung, die dazu ausgebildet ist, den Referenzwert $MG_{Xref}$ einer Messgröße $MG_X$ durch Mittelwertbildung über alle in einer Kalibrierungsphase erfassten Messgrößen $MG_X$ einer physikalischen Größe zu ermitteln.

7. Verfahren zum Ermitteln des Hydrationszustandes eines Köpers eines Menschen und/oder eines Säugetiers, aufweisend die folgenden Schritte:

- Senden zumindest von Mikrowellen in den Körper mittels einer ersten auf der Haut des Körpers angeordneten ersten Sensoranordnung (110);
- Ermitteln der vom Körper reflektierten und/oder transmittierten Mikrowellen mittels der ersten Sensoranordnung als erste Messgröße;
- Ermitteln einer zweiten Messgröße des Körpers mittels einer zweiten Sensoranordnung (104, 106, 110, 112, 114, 116, 118, 120, 122, 124); und
- Ermitteln des Hydrationszustandes des Körpers auf Grundlage der ersten und der zweiten Messgröße,

wobei die erste Sensoranordnung (110) die Dämpfung und/oder die Phasenverschiebung der Mikrowellen bei einer Resonanzfrequenz des Bereichs des Körpers ermittelt, in dem sich die Mikrowellen von der ersten Sensoranordnung ausbreiten.

8. Verfahren nach Anspruch 7, ferner aufweisend den folgenden Schritt:

- Erfassen der Impedanz des Körpers.

9. Verfahren nach Anspruch 7 oder 8, ferner aufweisend folgenden Schritt:

- Ermitteln zumindest einer weiteren Messgröße des Körpers mittels zumindest einer weiteren Sensoranordnung (104, 106, 110, 112, 114, 116, 118, 120, 122, 124); und
- Ermitteln des Hydrationszustandes des Körpers auf Grundlage der ersten und der zweiten Messgröße sowie der zumindest einen weiteren Messgröße.

10. Verfahren nach einem der Ansprüche 7 bis 9, wobei der Schritt des Ermittelns des Hydrationszustandes mit zumindest einer der folgenden Formeln durchgeführt wird:

$$TBW = K_0 + K_1 \times f_1(MG_1, MG_{1ref}) + \ldots + f_n(MG_n, MG_{nref});$$

oder

$$TBW = K_1 + K_2 \times MG_1/MG_{1ref} + K_3 \times MG_2/MG_{2ref};$$

oder

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref} \ldots;$$

wobei gilt:

TBW: $m_{Wasser}/m_{Körper}$;
MGx: Messgröße x;
$MG_{xref}$: Referenzwert der Messgröße x;
$f_1(MG_1, MG_{1ref})$ eine beliebige Funktion der Messgröße 1 und deren Referenzwert ist; und
$f_n(MG_n, MG_{nref})$ eine beliebige Funktion der Messgröße n und deren Referenzwert ist.

11. Verfahren nach Anspruch 10, ferner aufweisend die folgenden Schritte:

- Ermitteln einer Mehrzahl Werte der Messgrößen über einen vorbestimmten Zeitraum; und
- Ermitteln des Referenzwertes einer Messgröße durch Ermitteln eines Durchschnittswertes der jeweiligen Messgröße auf Grundlage der im vorbestimmten Zeitraum ermittelten Werte der jeweiligen Messgröße.

12. Computerprogrammprodukt, das, wenn es in einen Speicher eines Computers mit einem Prozessor, welcher eine Vorrichtung gemäß Anspruch 1 steuert, geladen wird, die Vorrichtung derart steuert, dass diese das Verfahren nach einem der Ansprüche 7 bis 11 durchführt.

**Claims**

1. A hydration state detection apparatus (100, 200, 300, 400) adapted to detect the hydration state of a body of a human or a mammal:

   - a first sensor arrangement (110, 304, 406) adapted to emit microwaves into the body via the skin and is adapted to detect microwaves transmitted by the body and/or microwaves reflected by the body, wherein the first sensor arrangement is adapted to be arranged on the skin of the body and wherein the first sensor arrangement outputs at least one first measurement variable which is based on the transmitted and/or reflected microwaves;
   - a second sensor arrangement (104, 106, 110, 112, 114, 116, 118, 120, 122, 124) adapted to measure at least one second physical variable of the body and to output a second measured variable which is based on the second physical variable; and
   - an evaluation device (102) adapted to determine the hydration state of the body based on the first measurement variable and the second measurement variable,

   wherein the first sensor arrangement is adapted to measure the phase shift and/or the attenuation of the microwaves at a resonance frequency of the region of the body in which the microwaves emitted by the first sensor arrangement propagate.

2. The hydration state detection apparatus (100, 200, 300, 400) according to claim 1, wherein the first sensor arrangement (110, 304, 406) is adapted to detect the impedance of the body.

3. The hydration state detection apparatus (100, 200, 300, 400) according to claim 1 or 2, having at least one further sensor arrangement (104, 106, 110, 112, 114, 116, 118, 120, 122, 124), each of which is adapted to measure at least one further physical variable of the body and which each output a further measurement variable which is based on the further physical variable, wherein the evaluation device is adapted to determine the hydration state of the body based on the first measurement variable, the second measurement variable and the further measurement variable.

4. The hydration state detection apparatus (100, 200, 300, 400) according to any one of claims 1 to 3, wherein the second and/or further sensor arrangement (104, 106, 110, 112, 114, 116, 118, 120, 122, 124) are adapted to measure in each case at least one of the following physical variables:

- a skin moisture;
- oxygen saturation;
- a pulse;
- a blood pressure;
- a body fat fraction.

5. The hydration state detection apparatus (100, 200, 300, 400) according to any one of claims 1 to 4, wherein the evaluation device is adapted to determine the hydration state of the body by means of at least one of the following formulae:

$$TBW = K_0 + K_1 \times f_1(MG_1, MG_{1ref}) + \ldots + f_n(MG_n, MG_{nref});$$

or

$$TBW = K_1 + K_2 \times MG_1/MG_{1ref} + K_3 \times MG_2/MG_{2ref};$$

or

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref} \ldots;$$

wherein:

TBW: $m_{Water}/m_{Body}$;
$MG_x$: measured variable x;
$MG_{xRef}$: reference value of the measured variable x;
$f_1(MG_1, MG_{1ref})$ is an arbitrary function of the measured variable 1 and its reference value; and
$f_n(MG_n, MG_{nref})$ is an arbitrary function of the measured variable n and the reference value thereof.

6. The hydration state detection apparatus according to any one of claims 1 to 5, further comprising a calibration device adapted to determine the reference value $MG_{xref}$ of a measurement variable $MG_x$ by averaging over all measurement variables $MG_x$ of a physical variable detected in a calibration phase.

7. A method for determining the hydration state of a body of a human and/or a mammal, comprising the following steps :

- transmitting at least microwaves into the body by means of a first sensor arrangement (110);
- determining the microwaves reflected and/or transmitted by the body by means of the first sensor arrangement as a first measurement variable;
- determining a second measurement variable of the body by means of a second sensor arrangement (104, 106, 110, 112, 114, 116, 118, 120, 122, 124); and
- determining the hydration state of the body on the basis of the first and the second measurement variable,

wherein the first sensor arrangement (110) determines the damping and/or the phase shift of the microwaves at a resonance frequency of the region of the body in which the microwaves propagate from the first sensor arrangement.

8.  The method of claim 7, further comprising the following step:

- determining the impedance of the body.

9.  The method according to claim 7 or 8, further comprising the following step:

- determining at least one further measurement variable of the body by means of at least one further sensor arrangement (104, 106, 110, 112, 114, 116, 118, 120, 122, 124); and
- determining the hydration state of the body on the basis of the first and the second measurement variable and the at least one further measurement variable.

10. The method according to any one of claims 7 to 9, wherein the step of determining the hydration state is carried out using at least one of the following formulae:

$$TBW = K_0 + K_1 \times f_1(MG_1, MG_{1ref}) + \ldots + f_n(MG_n, MG_{nref});$$

or

$$TBW = K_1 + K_2 \times MG_1/MG_{1ref} + K_3 \times MG_2/MG_{2ref};$$

or

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref} \ldots;$$

wherein:

TBW: $m_{Water}/m_{Body}$;
$MG_x$: measured variable x;
$MG_{xRef}$: reference value of the measured variable x;
$f_1(MG_1, MG_{1ref})$ is an arbitrary function of the measured variable 1 and its reference value; and
$f_n(MG_n, MG_{nref})$ is an arbitrary function of the measured variable n and the reference value thereof.

11. The method of claim 10, further comprising the steps of:

- determining a plurality of values of the measured variables over a predetermined period of time; and
- determining the reference value of a measurement variable by determining an average value of the respective measurement variable on the basis of the values of the respective measurement variable determined in the predetermined time period.

12. A computer program product which, when loaded into a memory of a computer having a processor controlling a device according to claim 1, controls the device to perform the method according to any one of claims 7 to 11.

**Revendications**

1.  Appareil de détection d'état d'hydratation (100, 200, 300, 400) adapté pour détecter l'état d'hydratation du corps d'un être humain ou d'un mammifère, comprenant :

- un premier agencement de capteur(s) (110, 304, 406) qui est adapté pour émettre des micro-ondes à l'intérieur du corps via la peau et qui est adapté pour détecter les micro-ondes qui sont transmises par le corps et/ou les micro-ondes qui sont réfléchies par le corps, dans lequel le premier agencement de capteur(s) est adapté pour être agencé sur la peau du corps et dans lequel le premier agencement de capteur(s) émet en sortie au moins une première variable de mesure qui est basée sur les micro-ondes transmises et/ou réfléchies ;
- un second agencement de capteur(s) (104, 106, 110, 112, 114, 116, 118, 120, 122, 124) qui est adapté pour mesurer au moins une seconde variable physique du corps et pour émettre en sortie une seconde variable mesurée qui est basée sur la seconde variable physique ; et
- un dispositif d'évaluation (102) qui est adapté pour déterminer l'état d'hydratation du corps sur la base de la première variable de mesure et de la seconde variable de mesure ;

dans lequel le premier agencement de capteur(s) est adapté pour mesurer le déphasage et/ou l'atténuation des micro-ondes à une fréquence de résonance de la région du corps à l'intérieur de laquelle les micro-ondes qui sont émises par le premier agencement de capteur(s) se propagent.

2. Appareil de détection d'état d'hydratation (100, 200, 300, 400) selon la revendication 1, dans lequel le premier agencement de capteur(s) (110, 304, 406) est adapté pour détecter l'impédance du corps.

3. Appareil de détection d'état d'hydratation (100, 200, 300, 400) selon la revendication 1 ou 2, comportant au moins un autre agencement de capteur(s) (104, 106, 110, 112, 114, 116, 118, 120, 122, 124), dont chacun est adapté pour mesurer au moins une autre variable physique du corps et dont chacun émet en sortie une autre variable de mesure qui est basée sur l'autre variable physique, dans lequel le dispositif d'évaluation est adapté pour déterminer l'état d'hydratation du corps sur la base de la première variable de mesure, de la seconde variable de mesure et de l'autre variable de mesure.

4. Appareil de détection d'état d'hydratation (100, 200, 300, 400) selon l'une quelconque des revendications 1 à 3, dans lequel le second et/ou l'autre agencement de capteur(s) (104, 106, 110, 112, 114, 116, 118, 120, 122, 124) sont/est adapté(s) pour mesurer dans chaque cas au moins l'une des variables physiques qui suivent :

- un taux d'hydratation de la peau ;
- une saturation en oxygène ;
- un pouls ;
- une pression sanguine ; et
- une proportion de tissus adipeux.

5. Appareil de détection d'état d'hydratation (100, 200, 300, 400) selon l'une quelconque des revendications 1 à 4, dans lequel le dispositif d'évaluation est adapté pour déterminer l'état d'hydratation du corps au moyen d'au moins l'une des formules qui suivent :

$$TBW = K_0 + K_1 \times f_1(MG_1, MG_{1ref}) + ... + f_n(MG_n, MG_{nref}) \; ;$$

ou

$$TBW = K_1 + K_2 \times MG_1/MG_{1ref} + K_3 \times MG_2/MG_{2ref} \; ;$$

ou

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref}) + ... \; ;$$

dans lesquelles :

$TBW : m_{eau}/m_{corps} \; ;$
$MG_x :$ variable mesurée x ;

$MG_{xref}$ : valeur de référence de la variable mesurée x ;
$f_1(MG_1, MG_{1ref})$ est une fonction arbitraire de la variable mesurée 1 et de sa valeur de référence ; et
$f_n(MG_n, MG_{nref})$ est une fonction arbitraire de la variable mesurée n et de sa valeur de référence.

6. Appareil de détection d'état d'hydratation selon l'une quelconque des revendications 1 à 5, comprenant en outre un dispositif d'étalonnage qui est adapté pour déterminer la valeur de référence $MG_{xref}$ d'une variable de mesure $MG_x$ en calculant la moyenne sur toutes les variables de mesure $MG_x$ d'une variable physique qui sont détectées lors d'une phase d'étalonnage.

7. Procédé pour déterminer l'état d'hydratation d'un corps d'un être humain et/ou d'un mammifère, comprenant les étapes qui suivent :

- la transmission d'au moins des micro-ondes à l'intérieur du corps au moyen d'un premier agencement de capteur(s) (110) ;
- la détermination des micro-ondes qui sont réfléchies et/ou qui sont transmises par le corps au moyen du premier agencement de capteur(s) en tant que première variable de mesure ;
- la détermination d'une seconde variable de mesure du corps au moyen d'un second agencement de capteur(s) (104, 106, 110, 112, 114, 116, 118, 120, 122, 124) ; et
- la détermination de l'état d'hydratation du corps sur la base des première et seconde variables de mesure ;

dans lequel le premier agencement de capteur(s) (110) détermine l'amortissement et/ou le déphasage des micro-ondes à une fréquence de résonance de la région du corps à l'intérieur de laquelle les micro-ondes se propagent depuis le premier agencement de capteur(s).

8. Procédé selon la revendication 7, comprenant en outre l'étape qui suit :

- la détermination de l'impédance du corps.

9. Procédé selon la revendication 7 ou 8, comprenant en outre les étapes qui suivent :

- la détermination d'au moins une autre variable de mesure du corps au moyen d'au moins un autre agencement de capteur(s) (104, 106, 110, 112, 114, 116, 118, 120, 122, 124) ; et
- la détermination de l'état d'hydratation du corps sur la base des première et seconde variables de mesure et de l'au moins une autre variable de mesure.

10. Procédé selon l'une quelconque des revendications 7 à 9, dans lequel l'étape de détermination de l'état d'hydratation est mise en œuvre en utilisant au moins l'une des formules qui suivent :

$$TBW = K_0 + K_1 \times f_1(MG_1, MG_{1ref}) + ... + f_n(MG_n, MG_{nref}) ;$$

ou

$$TBW = K_1 + K_2 \times MG_1/MG_{1ref} + K_3 \times MG_2/MG_{2ref} ;$$

ou

$$TBW = K_1 + K_2 \times MG_1/MG_{1ref} + K_3 \times MG_2/MG_{2ref} + K_4 \times MG_3/MG_{3ref} + K_5 \times MG_4/MG_{4ref}) + ... ;$$

dans lesquelles :

$TBW$ : $m_{eau}/m_{corps}$ ;
$MG_x$ : variable mesurée x ;
$MG_{xref}$ : valeur de référence de la variable mesurée x ;

$f_1(MG_1, MG_{1ref})$ est une fonction arbitraire de la variable mesurée 1 et de sa valeur de référence ; et
$f_n(MG_n, MG_{nref})$ est une fonction arbitraire de la variable mesurée n et de sa valeur de référence.

11. Procédé selon la revendication 10, comprenant en outre les étapes qui suivent :

- la détermination d'une pluralité de valeurs des variables mesurées sur une période temporelle prédéterminée ; et
- la détermination de la valeur de référence d'une variable de mesure en déterminant une valeur moyenne de la variable de mesure respective sur la base des valeurs de la variable de mesure respective qui sont déterminées dans la période temporelle prédéterminée.

12. Progiciel qui, lorsqu'il est chargé à l'intérieur d'une mémoire d'un ordinateur qui comporte un processeur qui commande un dispositif selon la revendication 1, commande le dispositif pour réaliser le procédé selon l'une quelconque des revendications 7 à 11.

Fig. 1

100

122    120    118    116    104

| Temperatur sensor | Gyroskop-, Beschleuni gungs- sensor- modul | Luftfeuch- tigkeit-, Temperatur sensor | SpO2- Sensor | Akku- mulator |

102

Mikropro- zessor

108

| Bluetooth- modul | Hautleit- wertsensor | Pulssensor | Sensoran- ordnung | Lade- einrichtung |

124    112    114    110    106

Haut des Nutzers

126

EP 3 375 366 B1

Fig. 2

200

204

Smart Device für Anzeige
der Daten

208

Ampelanzeige

210

202

Schweißband

206

Ampel

Fig. 3

EP 3 375 366 B1

Fig. 4

EP 3 375 366 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7291190 B
- US 7033321 B
- DE 102014109549 A1
- US 20090043222 A1
- US 20150289820 A1
- DE 102008002520 A